# Europäisches Patentamt
# European Patent Office
# Office européen des brevets

(19)

(11) Numéro de publication: **0 061 074**
**B1**

(12) **FASCICULE DE BREVET EUROPEEN**

(45) Date de publication du fascicule du brevet:
**03.07.85**

(51) Int. Cl.⁴: **A 61 B 5/08, G 01 N 1/22**

(21) Numéro de dépôt: **82101928.8**

(22) Date de dépôt: **11.03.82**

(54) Appareil pour le prélèvement et l'analyse des aérosols atmosphériques absorbés par les êtres vivants.

(30) Priorité: **25.03.81 LU 83254**

(43) Date de publication de la demande:
**29.09.82 Bulletin 82/39**

(45) Mention de la délivrance du brevet:
**03.07.85 Bulletin 85/27**

(84) Etats contractants désignés:
**DE FR GB IT NL**

(73) Titulaire: **COMMUNAUTE EUROPEENNE DE L'ENERGIE ATOMIQUE (EURATOM), Bâtiment Jean Monnet Plateau du Kirchberg Boîte Postale 1907, L-1019 Luxembourg (LU)**

(72) Inventeur: **Schuykens, Danielle Marie, 19, Place Eugène Keym, B-1170 Bruxelles (BE)**
Inventeur: **Federico, Antonio, via Del Tasso, 7, I-21027 Ispra (Va) (IT)**

(74) Mandataire: **Wey, Hans-Heinrich, Dipl.-Ing. et al, Patentanwälte Müller-Börner & Wey Widenmayerstrasse 49, D-8000 München 22 (DE)**

(56) Documents cités:
**EP - A - 0 006 256**
**DE - A - 2 938 856**
**DE - B - 1 039 702**
**FR - A - 1 147 482**
**US - A - 3 457 787**

**Environmental Science and Technology, 9, 1975, P. 971-972; G. Desaedeleer**
**Health Physics, Pergamon Press, 1966, Vol. 12, p. 173-207**

Le dossier contient des informations techniques présentées postérieurement au dépôt de la demande et ne figurant pas dans le présent fascicule.

## Description

L'objet de la présente invention est la réalisation d'un appareil pour le prélèvement et l'analyse des aérosols atmosphériques.

Lorsque l'on veut réaliser un système de récolte des aérosols expirés, on se trouve confronté à de nombreux problèmes dus aux réactions physiques et chimiques subies par les aérosols durant leur trajet entre le site de prélèvement et le système de piégeage. Il faut limiter au mieux les pertes sur les parois de l'instrument et les changements de distribution granulométrique qui peuvent conduire à des erreurs systématiques sur l'interprétation du taux de déposition en fonction du diamètre des particules. Les différences que l'on trouve dans la littérature ne sont pas imputables seulement aux différences physiologiques et pathologiques de sujet à sujet mais également à la méthodologie utilisée (génération d'aérosols, types d'aérosols, préleveurs, . . .).

Nous proposons ici un système de prélèvement qui, en principe, doit faire subir aux échantillons d'air inhalés et aux échantillons exhalés le même traitement physique, ce qui, bien entendu, est la seule condition qui permette une comparaison objective des deux échantillons et donc une mesure exacte des taux de déposition dans les voies respiratoires.

Avant de décrire notre propre système de prélèvement, il serait intéressant, par comparaison, de présenter les principes qui guident les autres techniques d'investigation. Les premières recherches ont été basées sur l'étude de l'absorption par les voies respiratoires d'aérosols monodimensionnels créés par des générateurs. Il s'agit là bien sûr d'une technique artificielle qui ne reproduit certainement pas la réalité des particules aériennes, avec leurs propriétés et hygroscopicité, par exemple. G. DESAEDELEER, le premier, a imaginé de prélever des particules aériennes inhalées et exhalées grâce à deux impacteurs, préleveurs capables d'isoler les particules suivant différentes fractions granulométriques. Un appareil conforme au préambule de la revendication jointe a été décrit par cet auteur dans l'article Environmental Science and Technology 9, 1975, p. 971–972. D'autres chercheurs ont appliqué ce même principe mais sans veiller particulièrement à une stricte similitude des deux chaînes de prélèvement: la chaîne de prélèvement de l'air inhalé (ambiant) et la chaîne de prélèvement de l'air expiré. (cf. en particulier Akselsson et al, Ann. Occup. Hyg. 19, 1976, p. 225–238 et Annegarn et al, in Session of «Particulate Analysis and Sampling», American Institute of Chemical Engineer 86th National Meeting, Houston, Texas, April 1–5, 1979.

Le système mis au point est destiné à collecter des aérosols atmosphériques, donc non produits par des générateurs. Deux chaînes de prélèvement assurent, grâce à des impacteurs, l'analyse granulométrique des aérosols ambiants et expirés. Enfin, une similitude rigoureuse a été prévue dans les deux chaînes de manière à assurer les conditions identiques de prélèvement des deux échantillons d'aérosol: en particulier, on a veillé:

— à réduire au minimum les perturbations subies par les flux d'air échantillonné (coudes, constrictions, clapets, . . .),

— à assurer une même température et une même hygroscopicité (saturation à 34 °C) des deux échantillons d'air,

— à assurer une parfaite symétrie des deux chaînes (débits, volumes, flux, géométrie, . . .).

Les conduits d'air sont en métal de manière à supprimer tout effet électrostatique sur les particules aériennes.

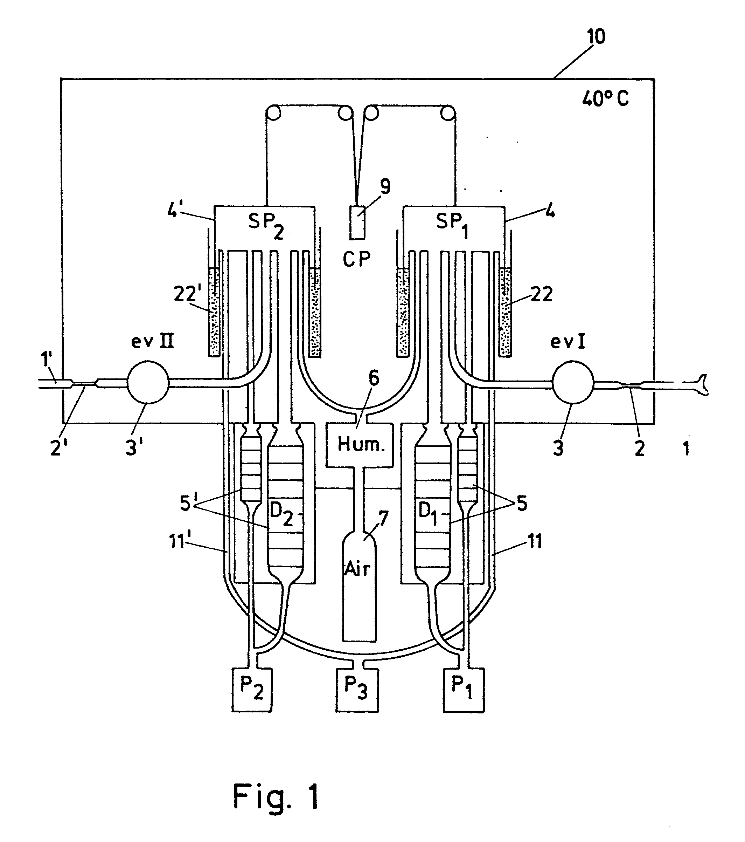

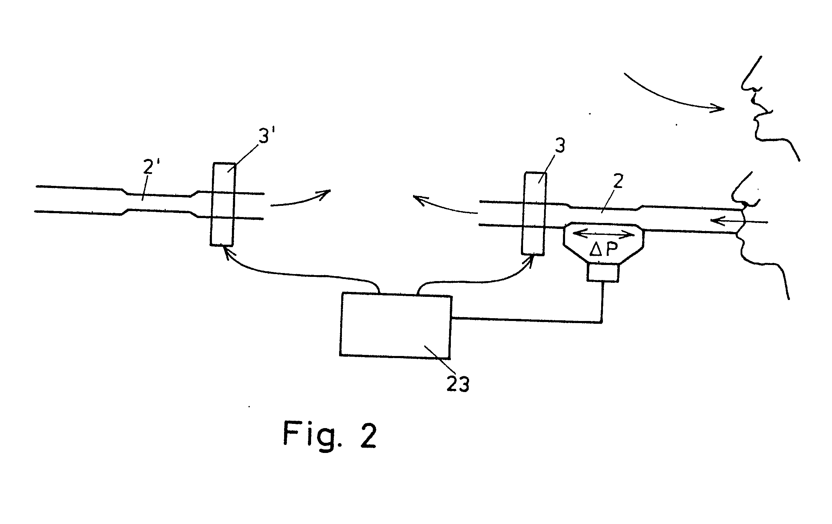

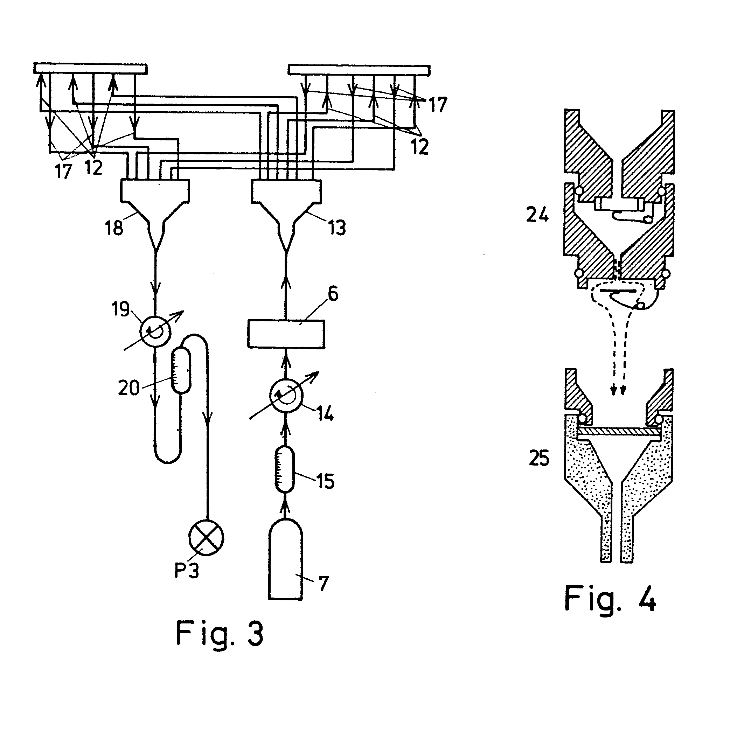

La figure 1 montre le schéma général de l'appareil et la figure 2, le système d'admission des échantillons d'air; la figure 3 se réfère à la régulation des débits d'air et la figure 4 montre les impacteurs en série.

La figure 1 reprend le schéma général du système; le reste de la description détaillera les diverses sections de l'appareil qui est de conception tout à fait symétrique.

Chacune des deux chaînes de prélèvement est constituée (dans l'ordre du passage des échantillons d'air. Les mêmes éléments des deux chaînes ont le même numéro de référence),

— d'une section d'admission de l'air avec un embout respiratoire 1 et un embout à l'air libre, 1'un tube de Poiseuille 2–2' qui commande l'ouverture ou la fermeture d'une électrovanne, 3–3',

— d'une section spirométrique (à cloche) 4–4' qui régularise le débit de l'air admis dans les impacteurs 5–5',

— de deux impacteurs travaillant en parallèle et destinés à prélever les particules suivant deux classes granulométriques (de 0,25 µm à 4 µm et de 0.50 µm à 16 µm),

— un humidificateur 6 porte l'humidité relative de l'air à la valeur imposée par le système respiratoire humain (saturation à 34 °C),

— un système d'admission d'air excédentaire alimenté par une bouteille d'air 7 comprimé fournit l'appoint d'air nécessaire pour garantir le débit nominal des impacteurs ($208 + 17,5 = 225$ cm$^3$/s ou $12,5 + 1,05 = 13,5/1$ min) au cas où le rythme respiratoire du sujet est insuffisant;

— au contraire, en cas d'expiration à rythme rapide, une pompe $P_3$ assure l'expulsion de l'air excédentaire: excès ou déficit d'air sont compensés par un jeu de vannes micrométriques commandées manuellement.

Les deux chaînes de prélèvement, symétriques par leur structure fonctionnent aussi de manière strictement synchrone:

— électrovannes asservies au même système de commande,

— spiromètres accouplés,

— débits strictement comparables, . . .

Lorsque le sujet exhale son volume d'air dans le système l'électrovanne à tiroir 3 s'ouvre automatiquement et l'air est pulsé dans le réservoir 4 d'où les particules sont prélevées par deux impacteurs à cascades 5 de type connu. La partie supérieure du spiromètre 4 s'élevant sous la pression causée par l'expiration du sujet entraîne, par un système de contrepoids CP 9 le second spiromètre 4' dans son mouvement ascendant. L'ouverture de 3 ayant entraîné électriquement l'ouverture de 3', la dépression causée dans la chaîne «Atmosphère» par le mouvement de la cloche pulse dans le réservoir 4' une quantité d'air égale à l'air exhalé par le sujet.

Les deux chaînes sont placées dans une enceinte 10 maintenue à une température supérieure à 35 °C afin d'éviter les pertes par condensation suivie de déposition sur les parois.

La chambre d'inhalation ainsi construite permet d'assurer au mieux un parallélisme rigoureux du comportement des particules dans les deux chaînes de prélèvement.

Afin d'éviter toute perturbation du flux d'air prélevé par l'appareil, on a renoncé à l'emploi de la valve à trois voies qui fait classiquement suite à l'embout bucal des chambres d'inhalation. On a donc opté pour un procédé plus exigeant pour le sujet soumis à l'examen mais plus fiable du point de vue récolte des aérosols. Une valve à trois voies eût permis au suject examiné d'inspirer et d'expirer l'air sans quitter l'embout bucal, ce qui est manifestement la solution la plus facile. Malheureusement, la valve à trois voies constitue un piège à particules incompatible avec les exigences imposées pour le fonctionnement de l'appareil; en outre, une telle vanne impose une pulsion en amont, elle ne peut donc être utilisée à l'entrée de la chaîne qui prélève l'air ambiant. Son emploi dans la chaîne d'expiration eût donc compromis le parallélisme strict entre les deux chaînes, qui est le but de l'invention.

On a préféré adopter un système actif qui impose au sujet d'inhalter l'air ambiant à bouche libre et d'insuffler l'air expiré à travers une électrovanne commandée par un senseur de flux. Outre qu'un système actif de ce genre perturbe bien moins l'écoulement de l'air, il permet également, par asservissement parallèle d'assurer simultanément la prise d'échantillon d'air ambiant par la deuxième chaîne. La figure 2 illustre le schéma du système d'admission des échantillons d'air.

Pour ouvrir automatiquement les électrovannes, on a utilisé un tube de Poiseuille. La variation de pression qu'il crée au passage du fluide est convertie en un signal électrique par un transducteur de pression. Le signal est mis en forme par un amplificateur qui, par l'intermédiaire d'une interface, commande l'ouverture simultanée des électrovannes, 3 et 3'.

Les impacteurs à cascade 5 et 5' fonctionnent sous un débit nominal strictement défini par le constructeur, débit qui garantit d'ailleurs l'analyse granulométrique des aérosols. Les spiromètres à cloche constituent le tampon nécessaire entre le débit intermittent des expirations et le débit régulier des impacteurs. Ils servent également de repère au réglage du débit moyen dirigé vers les impacteurs.

Le déplacement volumique maximum des cloches est de 6 580 cm$^3$. Le bouchon des cloches est constitué d'eau bidistillée 22. Le fond des spiromètres est amovible; c'est sur lui que viennent se connecter les embouts de l'adduction et d'évacuation de l'air 17 et 12 et du réglage des doseurs de débits moyens. En bas se fixe le porte-sonde hygrométrique qui permet de mesurer le degré hygrométrique dans le spiromètre en début et en fin d'expérience.

Le schéma reporté à la figure 3 illustre la régulation des débits d'air.

Un humidificateur 6 est relié à un répartiteur de flux 13 à six portes, connecté séquentiellement à une vanne micrométrique 14, un fluxmètre 15 et une bouteille d'air comprimé 7. Ces six portes sont reliées à six orifices 12 répartis trois à trois dans les deux spiromètres. Cet air de dilution sert non seulement à humidifier en continu ($\sim$ 33 cm$^3$/s ou $\sim$ 2 l/min) l'air contenu dans le section spirométrique mais aussi sert d'appoint en cas de volume d'expiration trop faible. L'expulsion d'air excédentaire s'effectue par les ($3 \times 2$) six orifices 17 restants et est réglée par un deuxième répartiteur de flux 18, une valve micrométrique 19, un fluxmètre 20 et une pompe à vide $P_3$.

Chaque spiromètre est relié à deux impacteurs (fig. 4) à cascade fe flux égal à (17,5 cm$^3$/s et 208 cm$^3$/s (1,05 l/min et 12,5 l/min) et dont les diamètres de coupure (= diamètre aérodynamique des particules dont l'efficience d'impaction est de 50%) sont égaux à > 4, 2, 1, 0.5, 0.25 et > 16, 8, 4, 2, 1, 0.5 µm respectivement. Les filtres finals recueillent respectivement les aérosols inférieurs à 0.25 µm et à 0.50 µm.

Les surfaces d'impaction 24 ont été recouvertes d'une membrane millipore qui reçoit les aérosols à analyser et les filtres finals 25 sont des membranes millipore de 0.4 µm de porosité. La chute de pression en aval des impacteurs est assurée par une pompe rotative à sec: $P_1$ et $P_2$. Chaque impacteur est relié au spiromètre par un tube d'Inox dont le diamètre est calculé de façon à égaliser la vitesse de l'air à l'intérieur des deux tubes et d'y garantir ainsi un comportement analogue des aérosols. Les impacteurs assurent un fractionnement des aérosols en fonction de leur diamètre aérodynamique, ce qui permet de confronter directement nos résultats avec le modèle du TASK LUNG GROUP qui fournit les données de déposition en fonction du diamètre aérodynamique moyen d'aérosols pour une distribution granulométrique log normale donnée (cf. Healta Physics, Pergamon Press 1966, Vol. 12, pages 173–207).

L'ensemble du système est enfermé dans une chambre 10 à double parois et isolée avec de la laine de verre. La thermostatisation est assurée par un pulseur d'air chaud connecté à un thermostat dont l'élément senseur est un thermocouple.

Un ventilateur pulse l'air chaud dans un tube fixé à trois parois de la chambre. Ce tube est muni d'une multitude de trous qui envoient l'air chaud horizontalement et verticalement, ce qui assure une climatisation uniforme de la chambre. Le thermocouple est fixé au centre de la chambre; il est relié à un thermostat à relais qui commande de ventilateur (220 V.A.). Trois sondes thermométriques sont fixées à des endroits différents afin de contrôler l'homogénéité de la température de la chambre. Chaque sonde thermométrique est reliée à un indicateur numérique de température muni d'une unité de communication prévu pour la sélection de 6 sondes thermométriques et de deux sondes hygrométriques (utilisées dans les spiromètres).

Il est normalement prévu que les impacteurs soient thermostatisés suivant le même principe. Cependant, pour des raisons de commodité, le système de chauffage a été simplifié: un rhéostat réglait l'intensité du courant traversant des bandes chauffantes enroulées autour des impacteurs. La température des impacteurs est maintenue à une valeur de 40 °C de manière à éviter la condensation de la vapeur d'eau suite à la détente adiabatique qui se produit dans les tuyères de l'appareil.

Le dosage des métaux déposés sur les surfaces d'impaction peut se réaliser par de nombreuses techniques analytiques pourvu qu'elles soient suffisamment sensibles: polarographie, colorimétrie, spectrométrie d'absorption atomique ou analyses par activation nucléaire (neutrons thermiques, protons) ou par activation électronique aux particules chargées (proton induced × Ray emission).

## Revendication

1. Appareil spirométrique pour le prélèvement et l'analyse des aérosols inhalés et expirés comportant une chaîne de prélèvement par expiration du sujet et une deuxième chaîne de prélèvement d'une atmosphère de références comprenant chacune une entrée (1, 1') des aérosols et un ensemble d'impacteurs (5,5') en parallèle, caractérisé par le fait que lesdites chaînes sont symétriques et comprennent chacune en outre un tube (2, 2') générateur de dépression, un transducteur de pression captant les variations de pression apparaissant dans le tube générateur de dépression placé dans la chaîne de prélèvement par expiration du sujet, une électrovanne (3, 3') commandée par le signal de sortie dudit transducteur de pression, un spiromètre (4, 4') à volume variable, les cloches mobiles des spiromètres étant reliées entre elles par une liaison à contrepoids de manière qu'un déplacement déterminé de la cloche mobile d'un spiromètre (4) entraîne un déplacement identique de celle de l'autre spiromètre (4'), l'ensemble d'impacteurs (5, 5') en parallèle étant alimenté par ledit spiromètre et asservi par des pompes de régulation de flux (P₁ et P₂), le débit d'air dans chaque ensemble d'impacteurs (5, 5') étant maintenu constant, côté entrée des spiromètres, par un répartiteur de flux (13) connecté séquentiellement à un humidificateur (6), une vanne micrométrique (14), un fluxmètre (15) et une bouteille (7) d'air comprimé et, côté sortie des spiromètres par un répartiteur de flux (18), connecté séquentiellement à une vanne micrométrique (19), un fluxmètre (20) et une pompe à vide (P₃).

## Patentanspruch

1. Spirometrisches Gerät zur Entnahme und Analyse von ein- und ausgeatmeten Aerosolen, bestehend aus einer Entnahmereihe für die Ausatmung des Lebewesens und einer zweiten Entnahmereihe für eine Bezugsatmosphäre, wobei jede Reihe einen Einlass (1, 1') für die Aerosole und eine parallel angeordnete Teilchenzähler-Einheit (5, 5') enthält, dadurch gekennzeichnet, dass die Reihen symmetrisch angeordnet sind und jede ausserdem ein Rohr (2, 2') für die Erzeugung eines Unterdruckes, einen Druckumwandler, der die in dem Unterdruckrohr, das sich in der Entnahmereihe für die Ausatmung des Lebewesens befindet, auftretenden Druckänderungen auffängt, ein durch das Ausgangssignal des Druckumwandlers betätigtes Magnetventil (3, 3') und ein Spirometer (4, 4') mit veränderlichem Volumen enthält, wobei die beweglichen Glocken der Spirometer durch eine Gegengewicht-Verbindung in der Art miteinander verbunden sind, dass eine bestimmte Verschiebung der beweglichen Glocke des einen Spirometers (4) eine identische Verschiebung der Glocke des anderen Spirometers (4') bewirkt, wobei die parallel angeordnete Teilchenzähler-Einheit (5, 5') durch den Spirometer gespeist und durch Strömungsregulierungspumpen (P₁ und P₂) zusammengedrückt wird und wobei der Luftausstoss in der Teilchenzähler-Einheit (5, 5') an der Einlasseite der Spirometer durch einen Strömungsverteiler (13), der aufeinanderfolgend mit einem Befeuchter (6), einem Mikrometerventil (14), einem Strömungsmessgerät (15) und einer Pressluftflasche (7) verbunden ist, und an der Auslasseite der Spirometer durch einen Strömungsverteiler (18), der aufeinanderfolgend mit einem Mikrometerventil (19), einem Strömungsmessgerät (20) und einer Vakuumpumpe (P₃) verbunden ist, konstant gehalten wird.

## Claim

1. Spirometric device for sampling and analyzing inhaled and exhaled aerosols, comprising a sampling train for the exhalation by the living being and a second sampling train for a reference atmosphere, wherein each train contains an inlet (1, 1') for the aerosols and an impinger (5, 5') parallel thereto, characterized in that the trains are symmetrically arranged and each contains a tube (2, 2') for creating a partial vacuum, a pressure converter absorbing the pressure changes occurring in the vacuum tube located in the sampling train for the exhalation of the living being, a magnetic valve (3, 3') actuated by the output signal of the pressure converter, and a spirometer (4, 4') with variable volume, wherein the movable bell jars of the spirometer are interconnected by a counterweight connection such that a certain displacement of the movable bell jar of the one spirometer (4) brings about an identical displacement of the bell jar of the other spirometer (4'), wherein the paralleldisposed impinger (5, 5') is fed by the spirometer and compressed by the flow-regulating pumps (P₁, P₂) and wherein the air expulsion in the impinger (5, 5') is kept constant at the inlet side of the spirometer by a flow distributer (13) with a humidifier (6), a micrometer valve(14), a

**0 061 074**

flow meter (15) and a compressed air tank (7) connected in series thereto, and at the outlet side of the spirometer by a flow distributer (18) with a micrometer'valve (19), a flow meter (20) and a vacuum pump ($P_3$) connected in series thereto.

*113*

Fig. 1

213

**Fig. 2**

Fig. 3

Fig. 4